Europäisches Patentamt

European Patent Office

Office européen des brevets

(19)

(11) Veröffentlichungsnummer : **0 398 397 B1**

(12) **EUROPÄISCHE PATENTSCHRIFT**

(45) Veröffentlichungstag der Patentschrift :
06.10.93 Patentblatt 93/40

(51) Int. Cl.⁵ : **A61J 1/06, A61M 5/31**

(21) Anmeldenummer : 90113525.1

(22) Anmeldetag : 19.02.87

(54) **Verwendung der Vorrichtung zur Applikation von Arzneimittelsuspensionen und Verfahren zur Herstellung einer homogenen Mischung dieser Suspension.**

(30) Priorität : 26.02.86 DE 3606163

(43) Veröffentlichungstag der Anmeldung :
22.11.90 Patentblatt 90/47

(45) Bekanntmachung des Hinweises auf die
Patenterteilung :
06.10.93 Patentblatt 93/40

(84) Benannte Vertragsstaaten :
AT BE CH DE ES FR GB GR IT LI LU NL SE

(56) Entgegenhaltungen :
US-A- 2 688 966
US-A- 3 045 494
US-A- 3 604 417

(60) Veröffentlichungsnummer der früheren
Anmeldung nach Art. 76 EPü : 0 235 691

(73) Patentinhaber : HOECHST
AKTIENGESELLSCHAFT
D-65926 Frankfurt (DE)

(72) Erfinder : Grau, Ulrich, Dr.
Am Forsthaus 1
D-65719 Hofheim am Taunus (DE)
Erfinder : Pohler, Wolfgang, Dr.
Martin-Wohmann Strasse 27
D-65719 Hofheim am Taunus (DE)

(74) Vertreter : VOSSIUS & PARTNER
Postfach 86 07 67
D-81634 München (DE)

## Beschreibung

Die Erfindung betrifft eine Verwendung eines zylindrischen Einkompartiment-Behälters zum Homogenisieren von Medikamentensuspensionen sowie ein Verfahren zur Herstellung einer homogenen Mischung dieser Suspension

Bekannt sind tragbare Infusionsgeräte zur automatischen Abgabe von Flüssigkeiten, insbesondere Insulin, wobei diese Flüssigkeiten in auswechselbaren Spritzampullen enthalten sind (Europäische Patentanmeldung mit der Veröffentlichungsnummer 0 143 895 und darin genannte Vorrichtungen). Die US 2688966 beschreibt kompartimentierte Ampullen, wobei ein Kompartiment ein Medikament zu trockener Form enthält und ein zweites, von dem ersten durch einen Stopfen getrenntes Kompartiment, das Lösungsmittel für das trockene Medikament enthält. Der zwischen dem Kompartimenten befindliche Stopfen ist durchbohrt und enthält eine Kugel, die vor Benutzung der Vorrichtung aus diesem herausgedrückt wird. Auch für das Lösungsmittelkompartiment ist eine Kugel vorgesehen, die eventuell vorhandene Präzipitaten wieder in Lösung bringen soll. Bekannt sind weiterhin tragbare Mehrfach-Injektionsgeräte, sogenannte "Pens" (EP-A- 0 058 536, WO 82/02662). Diese bekannten Vorrichtungen, die zur Mehrfachapplikation der Arzneimittel bestimmt sind, eignen sich jedoch nur für homogene Flüssigkeiten, insbesondere Lösungen. Bei dispersen Systemen wie Arzneimittelsuspensionen besteht nicht nur die Gefahr, daß es durch Absetzen der Kristalle zu mechanischen Schwierigkeiten kommt, sondern vor allem zu Fehldosierungen. Solche Fehldosierungen sind besonders dann kritisch, wenn es sich bei dem Medikament um ein hochwirksames Arzneimittel von geringer therapeutischer Breite handelt, wie beispielsweise Insulin.

Werden bei einer Insulinsuspension zu wenig Kristalle appliziert, weil die Suspension ungenügend aufgeschüttelt bzw. bereits wieder sedimentiert war, so ist eine Hyperglykämie mit den bekannten begleitenden Symptonen und mit erhöhtem Risiko zu Spätkomplikationen zu erwarten. Wird umgekehrt zu viel des Wirkstoffs appliziert, so ist eine Hyperinsulinämie und eine Hypoglykämie mit der begleitenden schweren Symptomatik, wie Schweißausbrüche, Krämpfen Bewußtlosigkeit bis hin zum Tod durch hyperglykämischen Schock zu befürchten.

Arzneimittelsuspensionen sind normalerweise in Behältnisse abgefüllt, die über der Suspension auch einen Gasraum enthalten. Die Suspension läßt sich dann dadurch homogenisieren, daß dieses Behältnis gerollt, auf den Kopf gedreht oder geschüttelt wird. Dafür verantwortlich ist die Präsenz der Gasblase und deren Beweglichkeit. Ist dagegen die Suspension im wesentlichen gasraumfrei abgefüllt, beispielsweise in Zylinderampullen, aus denen mehrfach appliziert werden soll, ist eine homogene Vermischung in angemessener Zeit normalerweise nicht möglich. Die physikalische Grundlage für dieses Phänomen liegt darin, daß die Dichte von Kristallen im allgemeinen sehr ähnlich der Dichte des Mediums ist.

Bei Dosiervorrichtungen, in denen aus einem Vorrat mittels einer Kolbenbewegung mehrfach Dosierungen appliziert werden sollen, ist jedoch eine im wesentlichen gasblasenfreie Abfüllung notwendig, da nur so eine reproduzierbare Dosierung gewährleistet ist. "Im wesentlichen gasblasenfrei" soll hierbei nicht ausschliessen, daß anfangs, also beispielsweise in der Ausgangsampulle, eine mehr oder weniger große Gasmenge vorhanden ist, die aber vor der ersten Applikation in bekannter Weise entfernt wird, so daß dann die Flüssigkeit praktisch gasblasenfrei ist. Gasblasenfreie Kristallsuspensionen können jedoch nicht durch Schütteln in einem vertretbaren Zeitraum in die nötige homogene Form gebracht werden. Hier ist insbesondere zu berücksichtigen, daß eine solche Homogenisierung auch alten und behinderten Patienten möglich sein muß. Aufgabe der Erfindung war es daher, für Infusions- bzw. Injektionsgeräte geeignete Vorratsbehältnisse zur Verfügung zu stellen, in denen Arzneimittelsuspensionen auch in Abwesenheit einer Gasblase homogen aufgeschüttelt werden können.

Erschwerend kommt hinzu, daß die für eine parenterale Applikation notwendige Sterilität des Inhalts zu gewährleisten ist. Das Vorratsbehältnis muß also so beschaffen sein, daß es in gefülltem Zustand mit Hilfe der üblichen Verfahren sterilisiert oder nach Sterilisation der Einzelteile unter aseptischen Bedingungen montiert, mit steriler Arzneimittelsuspension befüllt und verschlossen werden kann.

Es ist bekannt, daß Schmiermittel-Dispersionen und Lacke, die in Sprühdosen abgefüllt sind, als Mischkörper Stahlkugeln enthalten. Bei den Schmiermitteln handelt es sich jedoch um leicht dispergierbare Stoffe wie Molybdänsulfid und Graphit, die durch wenige Schüttelbewegungen leicht in eine sprühfähige Form gebracht werden können. Bei Lacken liegt demgegenüber ein relativ viskoses System vor, so daß beim Schütteln hohe Scherkräfte auftreten, die die Redispergierung erleichtern. Im übrigen enthalten diese Systeme große Mengen Treibgas, so daß diese Art der Durchmischung nicht ohne weiteres auf Arzneimittelsuspensionen übertragbar ist. Außerdem entsteht durch das Reiben der Stahlkugeln an der Wand des Behälters ein Metallabrieb, der jedoch bei diesen technischen Systemen nicht stört. Es besteht auch nicht die Gefahr einer Rekristallisation und damit einer Partikelvergröberung, da die dispergierten Schmierstoffe oder Pigmentpartikel im Applikationsmedium völlig oder doch praktisch unlöslich sind.

Aufgrund des fehlenden Gasraums und - insbesondere bei Zylinderampullen - aufgrund des ungünstigen Verhältnisses von Durchmesser zu Länge, das sich zudem während der Anwendung beim Vorschieben des Kolbens ständig verändert, war nicht zu erwarten, daß einfach Mischkörper, wie sie von Sprühdosen bekannt sind, auch bei in übliche Arzneimittelbehältnisse gasblasenfrei gefüllten Arzneimittelsuspensionen zur Homogenisierung geeignet sein könnten.

Zudem ist von vielen Arzneistoffkristallen - insbesondere vom Insulin - bekannt, daß sie empfindlich gegenüber mechanischer Belastung sind. Rührt man z.B. eine Insulin-Kristallsuspension in einem Becherglas auf einem üblichen Magnetrührer, so wird ein beträchtlicher Teil der Insulinkristalle aufgrund der Einwirkung des Magnet-Rührstäbchens zerrieben. Durch die damit verbundene Ober flächenvergrößerung der Teilchen wird ihre Lösungsgeschwindigkeit und damit der Depoteffekt in unerwünschter Weise verändert. Eine solche Veränderung der Partikelgröße war auch bei Verwendung von Mischkörpern in Arzneimittelbehältnissen zu befürchten.

Es wurde nun überraschenderweise gefunden, daß auch Arzneimittelsuspensionen, vor allem gasblasenfrei abgefüllte, mit Hilfe von Mischkörpern leicht in eine homogene Suspension gebracht werden können, wenn der Mischkörper inert ist, eine geeignete Dimension und eine hinreichend unterschiedliche Dichte zur Arzneimittelsuspension aufweist. Alle genannten Parameter sind hierbei durch einfache Vorversuche leicht zu ermitteln. Eine hinreichend unterschiedliche Dichte ist gegeben, wenn die Dichte des Mischkörpers mindestens 10 %, vorzugsweise mindestens 50 %, insbesondere mindestens 100 % höher als die des suspendierten Arzneimittels ist.

Überraschenderweise werden Insulin-Kristallsuspensionen, wie sie in den weiter hinten angeführten Beispielen Verwendung fanden, unter den beschriebenen Bedingungen praktisch nicht in ihrer Partikelgröße verändert.

Da die Arzneimittelbehälter, vor allem Spritzampullen, beispielsweise übliche Zylinderampullen oder auch Zweikammer-Spritzen (z.B. Pharm. Ind. 46 (1984, Nr.3) 317), üblicherweise aus Glas bestehen, werden die Mischkörper vorteilhaft ebenfalls aus Glas gefertigt. Geeignet sind aber auch Kunststoffe, beispielsweise Polymere fluorierter Ethylene wie PTFE oder keramische bzw. metallische, gegebenenfalls beschichtete Körper.

Die Gestalt der Mischkörper kann durch entsprechende Vorversuche optimiert werden, wobei zweckmäßigerweise darauf geachtet wird, daß das Volumen der Mischkörper relativ zum Reservoir gering bleibt, so daß vergleichsweise kleine Toträume resultieren. Im allgemeinen eignen sich einfache Formen für die Mischkörper wie Zylinder oder vorzugsweise Kugeln, aber auch Formkörper mit turbulenzenerzeugender Oberflächengestaltung wie Kugeln mit Bohrungen oder andere kompliziertere Formen kommen in Betracht.

Unter "inert" wird ein Mischkörper verstanden, der weder chemisch noch physikalisch in störender Weise mit der Arzneimittelzubereitung in Wechselwirkung tritt. Es darf also weder eine chemische Veränderung noch eine physikalische Beeinträchtigung wie Adsorption oder Abrieb in nennenswertem Maße auftreten.

Im Sinne der Erfindung geeignet ist also ein System, das mit wenigen Kipp- oder Schüttelbewegungen eine homogene Aufschüttelung erlaubt.

In den folgenden Beispielen wird die Erfindung näher erläutert.

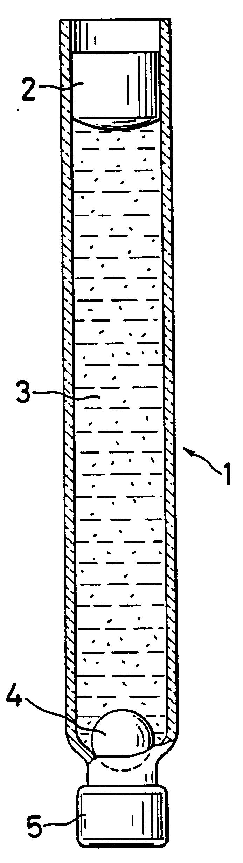

Die Figur zeigt eine erste Ausführungsform eines bei der Erfindung verwendeten zylindrischen Einkompartiment-Behälters im Längsschnitt.

In allen Beispielen und Vergleichsbeispielen wurden die in der Figur schematisch dargestellten Zylinderampullen (1) eingesetzt, die oben einen beweglichen Kolben (2) aufweisen, auf den eine (nicht dargestellte) Kolbenstange einwirken kann. Die Ampulle ist blasenfrei mit der Arzneimittelsuspension (3) gefüllt und enthält einen oder mehrere Mischkörper (4). In der Figur ist als Mischkörper eine Kugel dargestellt. Das Auslaßstück (5) ist in bekannter Weise verschlossen.

Als Arzneimittelsuspension dient eine wäßrige Insulin-Depotzubereitung (Basal H Insulin U-100 Hoechst[R]), die pro $\mu\ell$ 0,1 I.E . Insulin enthält. Die Aufschüttelbarkeit des Sediments wurde nach zwölfstündiger Standzeit (kopfstehend) durch Kippbewegungen von 180° visuell beurteilt.

In Vergleichsbeispiel 1 bis 3 und Beispiel 1 bis 4 hatten die Zylinderampullen (1) eine Länge von 62,3 mm und einen Innen-Durchmesser von 6,85 mm.

Vergleichsbeispiel 1:

Kein Glasperlenzusatz

Beurteilung: Homogene Aufschüttelung durch einfache Kippbewegungen in akzeptablem Zeitraum nicht möglich.

Vergleichsbeispiel 2:

Zusatz einer Glasperle von 3 mm Durchmesser.
Beurteilung: Aufschüttelbarkeit nicht reproduzierbar. Sediment gelangt mit einer Kugel in den Auslaßteil der Zylinderampulle und fixiert die Kugel dort.

Vergleichsbeispiel 3:

Zusatz von 2 Glasperlen, Durchmesser 3 mm.
Beurteilung: Wie bei Vergleichsbeispiel 2.

Beispiel 1:

Zusatz einer Glasperle vom Durchmesser 4 mm.
Beurteilung: Homogene Aufschüttelung durch 6 bis 9 Kippbewegungen.

Beispiel 2:

Zusatz von 2 Glasperlen, Durchmesser 4 mm.
Beurteilung: Wie bei Beispiel 1.

Beispiel 3:

Zusatz einer Glasperle vom Durchmesser 5 mm.
Beurteilung: Homogene Aufschüttelung durch 3 bis 4 Kippbewegungen.

Beispiel 4:

Zusatz einer Glasperle von 6 mm Durchmesser.
Beurteilung: Wie bei Beispiel 3.

Beispiele 5 und 6:

Zylinderampullen von 58 mm Länge und 6,85 mm Innen-Durchmesser, die eine Glaskugel (Durchmesser 5 mm) enthielten, wurden gasblasenfrei mit der Insulin-Suspension gefüllt und in einen handelsüblichen Insulin-"Pen" eingesetzt.

Nach unterschiedlichen Standzeiten zwischen den Entnahmen wurde der Inhalt durch fünf 180°-Kippbewegungen resuspendiert und jeweils 40 μl, entspr. 4 I.E. Insulin, in fünf Probenröhrchen dosiert. Zur Ermittlung der Homogenität wurde der Gehalt der einzelnen Proben an Insulin analytisch bestimmt.

Der Mittelwert ($\bar{x}$) von jeweils fünf Proben und die relative Standardabweichung ($s_{rel}$) sind in der folgenden Tabelle aus zwei parallelen Versuchsserien mit zwei Insulin-"Pens" zusammengefaßt:

| Standzeit vor Resuspendierung | $\bar{x}$ (I.E.) | | $s_{rel}$ (%) | |
|---|---|---|---|---|
| | Beispiel 5 | Beispiel 6 | Beispiel 5 | Beispiel 6 |
| 6 Stunden | 4,4 | 3,9 | 11,1 | 5,6 |
| 72 Stunden | 4,1 | 4,0 | 8,4 | 1,2 |
| 6 Stunden | 3,9 | 4,0 | 2,3 | 3,3 |
| 16 Stunden | 3,7 | 4,0 | 4,9 | 5,7 |

**Patentansprüche**

1. Verwendung eines zylindrischen Einkompartiment -Behälters, der auf der einen Seite mit einem beweglichen Kolben (2) und auf der anderen Seite durch ein Auslaßstück (5) verschlossen ist, und mit einer Medikamentensuspension (3) im wesentlichen gasblasenfrei gefüllt ist sowie in der Suspension einen oder mehrere Mischkörper (4) enthält, die gegenüber der Suspension inert sind, eine von der Suspension hinreichend unterschiedliche Dichte haben, innerhalb der Vorrichtung immer frei beweglich sind und so dimensioniert sind, daß die Suspension durch wenige Schüttelbewegungen homogenisiert wird, ohne den arzneilichen Depoteffekt der Suspension negativ zu beeinflussen, zum Homogenisieren von Medikamentensuspensionen.

2. Verwendung nach Anspruch 1, dadurch gekennzeichnet, daß der oder die Mischkörper (4) kugelförmig sind.

3. Verwendung nach Anspruch 1 oder 2, dadurch gekennzeichnet, daß ein bis drei kugelförmige Mischkörper enthalten sind.

4. Verwendung nach einem oder mehreren der vorhergehenden Ansprüche, dadurch gekennzeichnet, daß die Dichte des Mischkörpers (4) mindestens 10 % höher als die der Suspension (3) ist.

5. Verwendung nach einem oder mehreren der vorhergehenden Ansprüche, dadurch gekennzeichnet, daß die Dichte des Mischkörpers (4) mindestens 50 % höher als die der Suspension (3) ist.

6. Verwendung nach einem oder mehreren der vorhergehenden Ansprüche, dadurch gekennzeichnet, daß die Dichte des Mischkörpers (4) mindestens 100 % höher als die der Suspension ist.

7. Verwendung nach einem oder mehreren der vorhergehenden Ansprüche, dadurch gekennzeichnet, daß der Mischkörper (4) aus Glas, Metall, Kunststoff oder Keramik besteht.

8. Verwendung nach Anspruch 7, dadurch gekennzeichnet, daß der Mischkörper beschichtet ist.

9. Verwendung nach einem oder mehreren der vorhergehenden Ansprüche, dadurch gekennzeichnet, daß die Suspension (3) eine Kristallsuspension ist.

10. Verwendung nach Anspruch 9, dadurch gekennzeichnet, daß die Suspension (3) eine Insulin-Kristallsuspension ist.

11. Verwendung nach Anspruch 10, dadurch gekennzeichnet, daß die Insulin-Kristallsuspension eine Insulin-Kristallsuspension mit arzneilichem Depoteffekt ist.

12. Verwendung der Vorrichtung gemäß einer oder mehrerer Ansprüche 1 bis 8 als Vorratsbehälter einer Medikamentensuspension (3) für Infusions- und Injektionsgeräte.

13. Verwendung nach Anspruch 12, dadurch gekennzeichnet, daß die Suspension (3) eine Insulin-Kristallsuspension ist.

14. Verwendung nach den Ansprüchen 12 oder 13, dadurch gekennzeichnet, daß die Suspension (3) eine Insulin-Kristallsuspension mit arzneilichem Depoteffekt ist.

15. Verfahren zur Herstellung einer homogenen Mischung einer Medikamentsuspension mit einem zylindrischen Einkompartiment -Behälter, der auf der einen Seite mit einem beweglichen Kolben (2) und auf der anderen Seite durch ein Auslaßstück (5) verschlossen ist, und mit einer Medikamentensuspen sion (3) im wesentlichen gasblasenfrei gefüllt ist sowie in der Suspension einen oder mehrere Mischkörper (4) enthält, die gegenüber der Suspension inert sind, eine von der Suspension hinreichend unterschiedliche Dichte haben, innerhalb der Vorrichtung immer frei beweglich sind und so dimensioniert sind, daß die Suspension durch wenige Schüttelbewegungen homogenisiert wird, ohne den arzneilichen Depoteffekt der Suspension negativ zu beeinflussen.

16. Verfahren nach Anspruch 15, dadurch gekennzeichnet, daß die Medikamentensuspension (3) eine Kristallsuspension ist.

**17.** Verfahren nach Anspruch 15 oder 16, dadurch gekennzeichnet, daß die Suspension (3) eine Insulin-Kristallsuspension ist.

**18.** Verfahren nach einem oder mehreren der Ansprüche 15 bis 17, dadurch gekennzeichnet, daß die Suspension (3) eine Insulin-Kristallsuspension mit arzneilichem Depoteffekt ist.

## Claims

**1.** The use of a cylindrical one-compartment container which is sealed at one side by a movable piston (2) and at the other side by an outlet piece (5), and is filled with a medicament suspension (3) so as to be essentially gas bubble-free and contains one or more mixing elements (4) in the suspension which are inert toward the suspension, have a density which is sufficiently different to that of the suspension, have permanent freedom of movement within the device, and the dimensions of which are such that the suspension is homogenized by means of few shaking movements, with no adverse effect on the pharmaceutical depot effect of the suspension, for homogenizing medicament suspensions.

**2.** The use as claimed in claim 1, wherein the mixing element or elements (4) are spherical.

**3.** The use as claimed in claim 1 or 2, wherein one to three spherical mixing elements are present.

**4.** The use as claimed in one or more of the previous claims, wherein the density of the mixing element (4) is at least 10% greater than that of the suspension (3).

**5.** The use as claimed in one or more of the previous claims, wherein the density of the mixing element (4) is at least 50% greater than that of the suspension (3).

**6.** The use as claimed in one or more of the previous claims, wherein the density of the mixing element (4) is at least 100% greater than that of the suspension.

**7.** The use as claimed in one or more of the previous claims, wherein the mixing element (4) comprises glass, metal, plastic or ceramic.

**8.** The device as claimed in claim 7, wherein the mixing element is coated.

**9.** The use as claimed in one or more of the previous claims, wherein the suspension (3) is a crystal suspension.

**10.** The use as claimed in claim 9, wherein the suspension (3) is an insulin crystal suspension.

**11.** The use as claimed in claim 10, wherein the insulin crystal suspension is an insulin crystal suspension with a pharmaceutical depot effect.

**12.** The use of the device of one or more of claims 1 to 8 as a combiner for storing a medicament suspension (3) for infusion and injection devices.

**13.** The use as claimed in claim 12, wherein the suspension (3) is an insulin crystal suspension.

**14.** The use as claimed in claim 12 or 13, wherein the suspension (3) is an insulin crystal suspension with a pharmaceutical depot effect.

**15.** A process for the preparation of a homogeneous mixture of a medicament suspension using a cylindrical one-compartment container which is sealed at one side by a movable piston (2) and at the other side by an outlet piece (5), and is filled with a medicament suspension (3) so as to be essentially gas bubble-free and contains one or more mixing elements (4) in the suspension which are inert toward the suspension, have a density which is sufficiently different to that of the suspension, have permanent freedom of movement within the device, and the dimensions of which are such that the suspension is homogenized by means of few shaking movements, with no adverse effect on the pharmaceutical depot effect of the suspension.

**16.** The process as claimed in claim 15, wherein the medicament suspension (3) is a crystal suspension.

**17.** The process as claimed in claim 15 or 16, wherein the suspension (3) is an insulin crystal suspension.

**18.** The process as claimed in one or more of claims 15 to 17, wherein the suspension (3) is an insulin crystal suspension with a pharmaceutical depot effect.

**Revendications**

**1.** Utilisation d'un récipient cylindrique monocompartiment fermé d'un côté par un piston mobile (2) et de l'autre côté par un goulot de sortie (5), et empli, essentiellement sans bulles de gaz, d'une suspension médicamenteuse (3) et contenant un ou plusieurs corps mélangeurs (4), qui sont inertes par rapport à la suspension, présentent une densité suffisamment différente de celle de la suspension, sont à tout moment librement mobiles à l'intérieur du dispositif et présentent des dimensions telles que la suspension puisse être homogénéisée par quelques mouvements d'agitation sans affecter de façon néfaste l'effet retard médicamenteux de la suspension, permettant l'homogénéisation de suspensions médicamenteuses.

**2.** Utilisation selon la revendication 1, caractérisée en ce que le ou les corps mélangeur(s) (4) est (sont) sphérique(s).

**3.** Utilisation selon l'une des revendications 1 ou 2, caractérisée en ce que le dispositif contient un à trois corps mélangeur(s) sphérique(s).

**4.** Utilisation selon l'une ou plusieurs des revendications précédentes, caractérisée en ce que la densité du corps mélangeur (4) est d'au moins 10 % supérieure à celle de la suspension (3).

**5.** Utilisation selon l'une ou plusieurs des revendications précédentes, caractérisée en ce que la densité du corps mélangeur (4) est d'au moins 50 % supérieure à celle de la suspension (3).

**6.** Utilisation selon l'une ou plusieurs des revendications précédentes, caractérisée en ce que la densité du corps mélangeur (4) est d'au moins 100 supérieure à celle de la suspension.

**7.** Utilisation selon l'une ou plusieurs des revendications précédentes, caractérisée en ce que le corps mélangeur (4) est constitué de verre, de métal, de matière synthétiques ou de céramique.

**8.** Utilisation selon la revendication 7, caractérisée en ce que le corps mélangeur est garni d'un revêtement.

**9.** Utilisation selon l'une ou plusieurs des revendications précédentes, caractérisée en ce que la suspension (3) est une suspension de cristaux.

**10.** Utilisation selon la revendication 9, caractérisée en ce que la suspension (3) est une suspension de cristaux d'insuline.

**11.** Utilisation selon la revendication 10, caractérisée en ce que la suspension de cristaux d'insuline est une suspension de cristaux d'insuline ayant un effet retard médicamenteux.

**12.** Utilisation du dispositif selon une ou plusieurs des revendications 1 à 8 en tant que récipient réservoir d'une suspension médicamenteuse (3) pour appareils de perfusion et d'injection.

**13.** Utilisation selon la revendication 12, caractérisée en ce que la suspension (3) est une suspension de cristaux d'insuline.

**14.** Utilisation selon l'une des revendications 12 ou 13, caractérisée en ce que la suspension (3) est une suspension de cristaux d'insuline ayant un effet retard médicamenteux.

**15.** Procédé de préparation d'un mélange homogène d'une suspension médicamenteuse à l'aide d'un récipient cylindrique monocompartiment fermé d'un coté par un piston mobile (2) et de l'autre côté par un goulot de sortie (5), empli, de façon pratiquement exempte de bulles de gaz, d'une suspension médicamenteuse (3), et contenant un ou plusieurs corps mélangeur(s) (4) dans la suspension, ces corps étant inertes par rapport à la suspension, présentant une densité suffisamment différente de celle de la suspension, étant à tout moment librement mobiles à l'intérieur du dispositif et présentant des dimensions telles que la suspension puisse être homogénéisée par quelques mouvements d'agitation, sans affecter de façon néfaste l'effet retard médicamenteux de la suspension.

16. Procédé selon la revendication 15, caractérisé en ce que la suspension médicamenteuse (3) est une suspension de cristaux.

17. Procédé selon la revendication 15 ou 16, caractérisé en ce que la suspension (3) est une suspension de cristaux d'insuline.

18. Procédé selon l'une ou plusieurs des revendications 15 à 17, caractérisé en ce que la suspension (3) est une suspension de cristaux d'insuline ayant un effet retard médicamenteux.